(19) Europäisches Patentamt
European Patent Office
Office européen des brevets

(11) Publication number: **0 040 958**
**B1**

(12) **EUROPEAN PATENT SPECIFICATION**

(45) Date of publication of patent specification: **14.03.84**

(51) Int. Cl.$^3$: **C 07 C 69/533**, C 07 C 67/27

(21) Application number: **81302258.9**

(22) Date of filing: **21.05.81**

(54) **Process for the synthesis of gamma-unsaturated carboxylic acid esters.**

(30) Priority: **26.05.80 AU 3716/80**

(43) Date of publication of application:
**02.12.81 Bulletin 81/48**

(45) Publication of the grant of the patent:
**14.03.84 Bulletin 84/11**

(84) Designated Contracting States:
**CH DE FR GB IT LI NL**

(56) References cited:
**GB - A - 1 520 444**

(73) Proprietor: **ICI AUSTRALIA LIMITED**
**ICI House 1 Nicholson Street P.O.Box 4311**
**Melbourne Victoria 3001 (AU)**

(72) Inventor: **McGarry, Errol James**
**3 Argyll Court**
**Eltham North Victoria, 3095 (AU)**
Inventor: **Conway, Richard John**
**Flat 8, 48 Davies Street**
**Brunswick Victoria, 3056 (AU)**
Inventor: **Cooke, Michael James**
**73 Tanner Street**
**Richmond Victoria, 3121 (AU)**

(74) Representative: **Bishop, Nigel Douglas et al,**
**Imperial Chemical Industries PLC Legal**
**Department: Patents PO Box 6**
**Welwyn Garden City Herts, AL7 1HD (GB)**

**EP 0 040 958 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the synthesis of gamma-unsaturated carboxylic acid esters

The invention concerns a process for the preparation of $\gamma$-unsaturated carboxylic acid esters.

$\gamma$-Unsaturated carboxylates are useful intermediates in the preparation of the acid moiety of insecticidal cyclopropane carboxylates which are known in the art as pyrethroid insecticides.

In 1970 W. S. Johnson et al, Journal of the American Chemical Society, *92*, 741 (1970), published a process for the preparation of $\gamma$-unsaturated carboxylates by heating a mixture of an allylic alcohol and a large molar excess (seven equivalents) of triethyl orthoacetate in the presence of a weak acid catalyst.

In Australian Patent Specification No. 491 852 K. Kondo et al disclose processes for the preparation of cyclopropanecarboxylate esters in which the Johnson reaction is used to prepare the intermediate $\gamma$-unsaturated carboxylates. The specification of this patent teaches that the reaction is preferably carried out in two stages in the presence of an acid catalyst and a 20 to 100% excess of the orthocarboxylate.

A later publication by K. Kondo et al ("Synthetic Pyrethroids", Ed. M. Elliot, Chapter 12 page 128, ACS Symposium Series 42, Washington DC 1977) discloses that if a 1 to 1 molar mixture of 3-methylbut-2-enol (prenol) and triethyl orthoacetate is reacted using the Johnson process, the by-product prenyl 3,3-dimethylpent-4-enoate is formed in ca 20% yield. The formation of the by-product in this yield in effect represents an ca 40% reduction in the yield of the required ethyl ester. This publication also reports that the best yields of ethyl 3,3-dimethylpent-4-enoate are obtained by the Johnson process when a mixture of prenol and a 2 to 3 fold molar excess of tri-ethyl orthoacetate is reacted and concludes that the maximum yield of the required ethyl ester can only be obtained when an excess amount of triethyl orthoacetate is used. Thus Example A of GB—A—1 520 444 indicates the necessity of using a 100% excess of ethyl orthoacetate.

While the Johnson process does give good yields of $\gamma$-unsaturated carboxylates when at least a 2 to 3 fold molar excess of the orthocarboxylate is used, the use of such a large excess of the orthocarboxylate is a significant disadvantage in the preparation of $\gamma$-unsaturated carboxylates on a large scale. The excess orthocarboxylate must be carefully removed from the reaction mixture by fractional distillation before the product can be isolated and in large scale preparations this operation takes a considerable length of time and as a result consumes relatively large amounts of energy.

In view of these serious disadvantages the reaction has now been closely studied in an attempt to reduce the amount of excess orthocarboxylate required while maintaining a high yield of the required $\gamma$-unsaturated carboxylate. As a result of these investigations it has now been found that high yields of $\gamma$-unsaturated carboxylates can be obtained by reacting an allylalcohol with only a relatively small excess of triethyl orthoacetate.

Accordingly the invention provides a process for the preparation of a $\gamma$-unsaturated carboxylate of the formula I

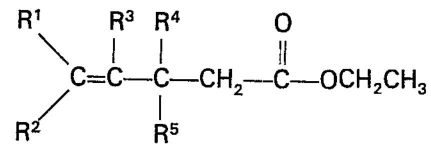 I

wherein $R^1$, $R^2$, $R^3$, $R^4$ and $R^5$ are independently chosen from hydrogen and $C_1$ to $C_6$ alkyl, which process comprises the reaction of an allyl alcohol of the formula II

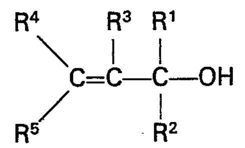 II

with triethyl orthoacetate in the presence of an acidic catalyst and wherein the triethyl orthoacetate is heated to a temperature in the range of from 130°C to 160°C and the allyl alcohol is slowly added to the hot triethyl orthoacetate.

The process of the invention may be used to particular advantage in the preparation of ethyl 3,3-dimethylpent-4-enoate by the reaction of 3-methylbut-2-enol (prenol) with triethyl orthoacetate.

The nature of the catalyst used in the process of the invention is not narrowly critical. A wide range of acid catalysts may be employed including inorganic acids such as phosphoric acid, hydrochloric acid and sulfuric acid; Lewis acids such as aluminium chloride, zinc chloride and ferric chloride; phenols such as phenol, alkylphenols (e.g. the cresols and xylenols), nitrophenols and the naphthols; aliphatic and aromatic carboxylic acids such as the $C_1$ to $C_6$ aliphatic carboxylic acids, cyclohexane carboxylic acids and benzoic acids; and aliphatic and aromatic sulfonic acids such as the methanesulfonic acids and the benzene—sulfonic acids. Preferred acidic catalysts include inorganic acids, Lewis acids and phenols. More preferred catalysts include orthophosphoric acid, aluminium chloride and phenol. Particularly preferred catalysts include aluminium chloride.

The amount of catalyst used in the process of the invention is not narrowly critical. Usually the amount of catalyst employed ranges from 1 to 10 mole percent of the allyl alcohol reactant.

In the process of the invention the triethyl orthoacetate is first heated to temperature close to its boiling point and the allyl alcohol is slowly added. The temperature to which the triethyl orthoacetate is heated is preferably in the range of from 130°C to 160°C, and more preferably from 140°C to 150°C, and the reaction mixture is maintained at this temperature during the addition.

Prior art processes teach that in order to prepare γ-unsaturated carboxylates in good yield by the reaction of an allyl alcohol and an orthocarboxylate, the orthocarboxylate must be present in a large excess. In general mole ratios of orthocarboxylate to allyl alcohol of the order of 3:1 and greater have been recommended. In contrast in the process of the present invention surprisingly good yields of the γ-unsaturated carboxylate can be obtained using mole ratios of orthocarboxylate to allyl alcohol in the range from 1:1 to 1.5:1. Higher ratios may be employed if desired, but in practice it has been found that in the process of the present invention, a mole ratio of orthocarboxylate to allyl alcohol of 1.2:1 gives a yield of γ-unsaturated carboxylate equal to or better than that obtained by prior art processes using a ratio of orthocarboxylate to allyl alcohol of at least 3:1.

A further advantage of the process of the present invention is the relatively short reaction time required even when the process is carried out on a relatively large scale. For example, the reaction has been carried out on a 40 mole scale by the slow addition of prenol over a period of 15 hours to a mixture of triethyl orthoacetate and aluminium chloride catalyst heated at a temperature of 140—150°C, to give ethyl 3,3-dimethylpent-4-enoate in a yield, after distillation, of 91%.

It will be evident to those skilled in the art that ethanol is formed as a by-product of the reaction. It follows that as the reaction presumably involves an initial interchange between the triethyl orthoacetate and the allyl alcohol to give a mixed orthoester and ethanol it is desirable to remove ethanol from the reaction mixture as it is formed to promote the production of the mixed orthoester. Thus in the process of the present invention it is preferred to equip the reaction vessel with a means to remove the relatively low boiling ethanol (b.p. 78°C) from the hot reaction mixture as it is formed during the addition of the allyl alcohol. This may be conveniently achieved by equipping the reaction vessel with a suitable still-head and continuous distilling the ethanol from the reaction mixture during the addition of the allyl alcohol and until the removal of ethanol from the reaction mixture is complete.

On completion of the reaction following the process of the present invention, the relatively small excess of triethyl orthoacetate may be removed from the reaction mixture either by distillation or by hydrolysis by water and removal of the hydrolysis products in the aqueous phase. The γ-unsaturated carboxylate product may then be purified by distillation if required.

The invention is now illustrated by but in no way limited to, the following Examples.

Example 1

A mixture of triethyl orthoacetate (194 g; 1.2 mole) and orthophosphoric acid (6 g; 0.06 mole) was heated with stirring to a temperature of 145°C. Prenol (86 g; 1.0 mole) was added dropwise to the stirred mixture over a period of 4 hours, during which time the temperature of the reaction mixture was maintained in the range from 140 to 150°C and ethanol was continuously removed from the reaction mixture by distillation. After completion of the addition the reaction mixture was maintained at a temperature of 150°C for a further 1 hour.

Water (50 ml) was added to the cooled reaction mixture and the aqueous mixture was heated at a temperature of 90°C for a period of 1 hour in order to hydrolyse the excess triethyl orthoacetate. The aqueous layer was then removed and the product was distilled to give ethyl 3,3-dimethylpent-4-enoate (124.5 g; 80%), b.p. 83—88°C/50 mm.

Example 2

The procedure described in Example 1 above was repeated with the exception that phenol (0.055 mole) was used as catalyst rather than phosphoric acid. Ethyl 3,3-dimethylpent-4-enoate was obtained in 75.3% yield.

Example 3

A mixture of triethyl orthoacetate (194 g; 1.2 mole) and anhydrous aluminium chloride (5.4 g; 0.04 mole) was heated with stirring to a temperature of 144°C. Prenol (86 g; 1.0 mole) was added dropwise to the stirred reaction mixture over a period of 5 hours. During the addition the reaction mixture was maintained at a temperature in the range from 144 to 150°C and ethanol was continuously removed from the reaction mixture by distillation. After completion of the addition the reaction mixture was distilled and the excess triethyl orthoacetate was recovered and then the product, ethyl 3,3-dimethylpent-4-enoate (143.8 g 92.2%) was collected, b.p. 80—85°C/50 mm.

Example 4

A mixture of triethyl orthoacetate (7.76 kg; 48 moles) and anhydrous aluminium chloride (216 g; 1.6 mole) was heated with stirring to a temperature of 145°C. Prenol (3.44 kg; 40 moles) was slowly added to the stirred reaction mixture over a period of 15 hours. During the addition the reaction mixture was maintained at a temperature in the range from 140 to 150°C and ethanol was continuously removed from the reaction mixture by distillation. After completion of the addition the reaction mixture

was distilled to recover the excess triethyl orthoacetate and then the product, ethyl 3,3-dimethylpent-4-enoate (5.66 kg; 91.0%), b.p 80—85°C/50 mm.

## Claims

1. A process for the preparation of a $\gamma$-unsaturated carboxylate of formula I

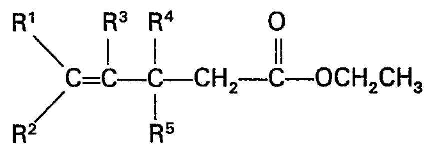
(I)

wherein $R^1$, $R^2$, $R^3$, $R^4$ and $R^5$ are independently chosen from hydrogen and $C_1$ to $C_6$ alkyl, which process comprises the reaction of an allyl alcohol of formula II

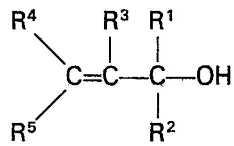
(II)

with triethyl orthoacetate characterised in that (a) an acidic catalyst is present (b) the triethyl orthoacetate is heated to a temperature in the range of from 130 to 160°C (c) the allyl alcohol is slowly added to the hot triethyl orthoacetate (d) ethanol is continuously distilled from the reaction mixture during the addition of the allyl alcohol and until the removal of the ethanol from the reaction mixture is complete, and (e) the mole ratio of the triethyl orthoacetate to the allyl alcohol of formula II is in the range of from 1:1 to 1.5:1.

2. A process according to claim 1 further characterised in that the triethyl orthoacetate is heated to a temperature in the range of from 140 to 150°C.

3. A process according to claim 1 further characterised in that the mole ratio of the triethyl orthoacetate to the fully alcohol is 1.2:1.

4. A process according to any one of claims 1 to 3 for the preparation of a compound of formula I further characterised in that $R^1$, $R^2$ and $R^3$ are hydrogen and $R^4$ and $R^5$ are each methyl.

5. A process according to claim 1 further characterised in that the acidic catalyst is an inorganic acid, Lewis acid, phenol, naphthol, aliphatic carboxylic acid, aromatic carboxylic acid, aliphatic sulfonic acid or aromatic sulfonic acid.

6. A process according to claim 5 further characterised in that the catalyst is chosen from phosphoric acid, hydrochloric acid, sulfuric acid, aluminium chloride, zinc chloride, ferric chloride, phenol, cresol, xylenol, nitrophenol, $\alpha$-naphthol, $\beta$-naphthol, the $C_1$ to $C_6$ aliphatic carboxylic acids, cyclohexane carboxylic acid, benzoic acid, the $C_1$ to $C_6$ aliphatic sulfonic acid and benzene sulfonic acid.

7. A process according to claim 6 further characterised in that the catalyst is chosen from orthophosphoric acid, aluminium chloride and phenol.

8. A process according to any one of claims 1 to 7 further characterised in that the amount of catalyst employed ranges from 1 to 10 mole percent of the allyl alcohol of formula II.

## Revendications

1. Procédé de préparation d'une carboxylate $\gamma$-insaturé de formule I

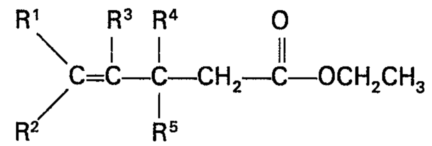
I

dans laquelle $R^1$, $R^2$, $R^3$, $R^4$ et $R^5$ sont choisis indépendamment entre l'hydrogène et des groupes alkyle en $C_1$ à $C_6$, procédé qui comprend la réaction d'un alcool allylique de formule II

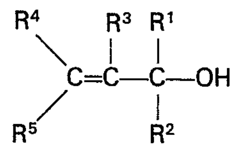
II

avec l'orthoacétate de triéthyle, caractérisé en ce que (a) un catalyseur acide est présent (b) l'orthoacétate de triéthyle est chauffé à une température comprise dans l'intervalle de 130 à 160°C, (c) l'alcool allylique est ajouté lentement à l'orthoacétate de triéthyle chaud, (d) de l'éthanol est continuellement chassé par distillation du mélange réactionnel pendant l'addition de l'alcool allylique et jusqu'à ce que l'élimination de l'éthanol du mélange réactionnel soit totale et (e) le rapport molaire de l'orthoacétate de triéthyle à l'alcool allylique de formule II se situe dans l'intervalle de 1:1 à 1,5:1.

2. Procédé suivant la revendication 1, caractérisé en outre en ce que l'orthoacétate de triéthyle est chauffé à une température comprise dans l'intervalle de 140 à 150°C.

3. Procédé suivanτ la revendication 1, caractérisé en outre en ce que le rapport molaire de l'orthoacétate de triéthyle à l'alcool allylique est égal à 1,2:1.

4. Procédé suivant l'une quelconque des revendications 1 à 3 pour la préparation d'un composé de formule I, caractérisé en outre en ce que $R^1$, $R^2$ et $R^3$ sont des atomes d'hydrogène et $R^4$ et $R^5$ représentent chacun un group méthyle.

5. Procédé suivant la revendication 1, caractérisé en outre en ce que le catalyseur acide est un acide inorganique, un acide de Lewis, le phénol, le naphthol, un acide carboxylique aliphatique, un acide carboxylique aromatique, un

acide sulfonique aliphatique ou un acide sulfonique aromatique.

6. Procédé suivant la revendication 5, caractérisé en outre en ce que le catalyseur est choisi entre l'acide phosphorique, l'acide chlorhydrique, l'acide sulfurique, le chlorure d'aluminium, le chlorure de zinc, le chlorure ferrique, le phénol, le crésol, le xylénol, le nitrophénol, l'$\alpha$-naphthol, le $\beta$-naphtol, les acides carboxyliques aliphatiques en $C_1$ à $C_6$, l'acide cyclohexanecarboxylique, l'acide benzoïque, les acides sulfoniques aliphatiques en $C_1$ à $C_6$ et l'acide benzènesulfonique.

7. Procédé suivant la revendication 6, caractérisé en outre en ce que le catalyseur est choisi entre l'acide orthophosphorque, le chlorure d'aluminium et le phénol.

8. Procédé suivant l'une quelconque des revendications 1 à 7, caractérisé en outre en ce que la quantité de catalyseur utilisé va de 1 à 10 moles % de l'alcool allylique de formule II.

**Patentansprüche**

1. Verfahren zur Herstellung eines $\gamma$-ungesättigten Carbonsäureesters der Formel I

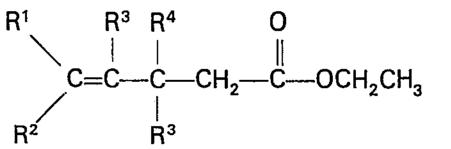

(I)

worin $R^1$, $R^2$, $R^3$, $R^4$ und $R^5$ unabhängig ausgewählt sind aus Wasserstoff und C—$C_6$-Alkyl, bei welchem Verfahren ein Allylalkohol der Formel II

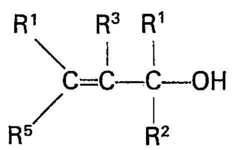

II

mit Triäthyl-orthoacetat umgesetzt wird, dadurch gekennzeichnet, daß (a) ein saurer Katalysator anwesend ist, (b), das Triäthylorthoacetat auf eine Temperatur im Berich von 130 bis 160°C erhitzt wird, (c) der Allyl-alkohol dem heißen Triäthyl-orthoacetat langsam zugegeben wird, (d) Äthanol kontinuierliche währen der Zugabe des Allylalkohols und bis zur vollständigen Entfernung des Äthanols aus dem Reaktionsgemisch abdestilliert wird und (e) das Molverhältnis des Triäthyl-orthoacetats zum Allylalkohol der Formel II im Bereich von 1:1 bis 1,5:1 liegt.

2. Verfahren nach Anspruch 1, dadurch gekennzeichnet, daß das Triäthyl-orthoacetat auf eine Temperatur im Bereich von 140 bis 150°C erhitzt wird.

3. Verfahren nach Anspruch 1, dadurch gekennzeichnet, daß das Molverhältnis des Triäthyl-orthoacetats zum Allylalkohol 1,2:1 beträgt.

4. Verfahren nach einem der Ansprüche 1 bis 3 zur Herstellung einer Verbindung der Formel I, dadurch gekennzeichnet, daß $R^1$, $R^2$ und $R^3$ für Wasserstoff stehen und $R^4$ und $R^5$ jeweils für Methyl stehen.

5. Verfahren nach Anspruch 1, dadurch gekennzeichnet, daß der sauer Katalysator aus einer anorganischen Säure, einer Lewis-Säure, einem Phenol, einem Naphthol, einer aliphatischen Carbonsäure, einer aromatischen Carbonsäure, einer aliphatischen Sulfonsäure oder einer aromatischen Sulfonsäure besteht.

6. Verfahren nach Anspruch 5, dadurch gekennzeichnet, daß der Katalysator ausgewählt wird aus Phosphorsäure, Salzsäure, Schwefelsäure, Aluminiumchlorid, Zinkchlorid, Eisen(III)-chlorid, Phenol, Cresol, Xylenol, Nitrophenol, $\alpha$-Naphthol, $\beta$-Naphthol den aliphatischen $C_1$—$C_6$-Carbonsäuren, Cyclohexancarbonsäure, Benzoesäure, den aliphatischen $C_1$—$C_6$-Sulfonsäuren und Benzolsulfonsäure.

7. Verfahren nach Anspruch 6, dadurch gekennzeichnet, daß der Katalysator ausgewählt wird aus Orthophosphorsäure, Aluminiumchlorid und Phenol.

8. Verfahren nach einem der Ansprüche 1 bis 7, dadurch gekennzeichnet, daß die verwendete Menge des Katalysators im Bereich von 1 bis 10 mol-%, bezogen auf den Allylalkohol der Formel II, liegt.